# EUROPEAN PATENT APPLICATION

(11) **EP 4 223 871 A1**
(43) Date of publication of application: **09.08.2023**
(21) Application number: 21875635.1
(22) Date of filing: 28.09.2021
(51) Int. Cl.: C12N 5/077, A61K 35/55, A61L 27/36, A61P 5/18, C12M 1/00, G01N 33/15, G01N 33/50

(54) **PARATHYROID GLAND CELLS**

(30) Priority: 29.09.2020 JP 2020162912
(71) Applicant: National University Corporation Tokyo Medical and Dental University, Tokyo 113-8510 (JP); Takeda Pharmaceutical Company Limited, Osaka-shi, Osaka 541-0045 (JP)
(72) Inventor: TAKEBE Takanori, Tokyo 113-8510 (JP); SAIKI Norikazu, Tokyo 113-8510 (JP)
(74) Representative: Jones, Nicholas Andrew
(86) International application number: PCT/JP2021/035681
(87) International publication number: WO 2022/071334

(57) **Abstract**

The present invention provides means for producing a parathyroid gland organoid. A culture method of the present invention includes a cell population containing a parathyroid gland cell in a ratio of 50% or more, and a mesenchymal cell in a ratio of 10% or more; and a step of culturing the cell population.

## Description

### Technical Field

The present invention relates to a cell population containing a parathyroid gland cell and a culture system therefor that can be used for producing a parathyroid gland organoid or a three-dimensional organ.

### [Background Art]

Aiming to develop medicaments for diseases of various organs and realize regenerative medicine, research and development have been conducted to produce a cell structure obtained by self-assembly of cultured cells to reproduce a three-dimensional structure of each organ or a complicated structure of a blood vessel, a bile duct or another vessel, namely, an organoid. As an advanced cell structure of such an organoid, an anlage of an organ formed at an early stage of development (an organ bud) can be produced by culturing a combination of specific types of cells including an undifferentiated cell. Such an organoid and the like that can be designated also as a "mini-organ" is, as compared with conventional cells and the like of an organ each singly cultured, highly useful because the organoid can be used as an evaluation system closer to a living body in evaluation of medicinal effect or toxicity, and in addition, can be used in a transplant operation or production of a plasma protein.

There are prior art literatures on an organoid of parathyroid gland or a similar cell aggregate as follows:
Non Patent Literature 1 describes that a spheroid was obtained by culturing a tonsil-derived mesenchymal stem cell (MSC) in a medium containing a prescribed component (such as cytokine) in a microwell, that the spheroid expressed parathyroid gland hormone (PTH) and N-cadherin at high level, and that the spheroid was transplanted in a rat having the parathyroid gland excised. It seems, based on a stained image, that the spheroid substantially contained only PTH-positive cells.
Non Patent Literature 2 describes that a spheroid was obtained by culturing, in a non-stick plastic, a parathyroid gland cell derived from a hyperparathyroidism patient, that the spheroid secretes PTH for a long period of time (150 days or more), and that the spheroid was transplanted in a mouse.
Non Patent Literature 3 describes that a parathyroid gland cell was produced by culturing an iPS cell in a medium containing a prescribed component (such as cytokine).

### Citation List

### Non Patent Literature

Non Patent Literature 1: Park et al., Scaffold-free parathyroid tissue engineering using tonsil-derived mesenchymal stem cells, Acta Biomaterialia, Volume 35, 15 April 2016, Pages 215-227
Non Patent Literature 2: Kanai et al., Suppression of parathyroid hormone production in vitro and in vivo by RNA interference, Kidney International (2009) 75, 490-498
Non Patent Literature 3: Derivation of Parathyroid Gland Cells and Their Progenitors from Induced Pluripotent Stem Cells (iPSCs) for Personalized Therapy, Institutional Biosafety Committee, Office of Research, University of California, San Francisco

### Summary of Invention

### Technical Problem

An object of the present invention is to provide means for producing a parathyroid gland organoid.

### Solution to Problem

The present inventors found that a parathyroid gland organoid can be obtained by culturing, in a medium containing a suitable component (such as cytokine), a cell population containing a parathyroid gland cell and a mesenchymal cell in prescribed ratios. Besides, it was found that an aggregate of a progenitor cell (anterior foregut) of a parathyroid gland cell obtained by culturing an iPS cell, and a cell present therearound (stromal cell) can be used respectively as the parathyroid gland cell and the mesenchymal cell used in this culture method, and that a cell population obtained by dissociating these cells once, and mixing the resultants in prescribed ratios is suitably used in the culture method. The present inventors have found that the above-described problem can be solved by such means, resulting in accomplishing the present invention.

Specifically, the present invention provides the following [1] to [18] :
[1] A cell population, comprising a parathyroid gland cell in a ratio of 50% or more, and a mesenchymal cell in a ratio of 10% or more.
[1A] A cell population, comprising a parathyroid gland cell in a ratio of 50% or more, and a mesenchymal cell in a ratio of 5% or more.
[2] The cell population according to item 1, further comprising a vascular endothelial cell.
[3] A matrix composition, comprising a matrix, and the cell population according to item 1 in a state embedded in the matrix.
[4] A culture system, comprising the cell population according to item 1 or the matrix composition according to item 3, a medium for culturing the cell population, and a culture vessel.
[5] The culture system according to item 4, wherein the culture vessel is a round bottom plate.
[5A] The culture system according to item 4, wherein the culture vessel is a vessel for suspension culture, stirred-suspension culture, or channel culture.
[6] The culture system according to item 4, wherein the medium contains two or more components selected from the group consisting of SHH, FGF8, FGF10, Wnt3a, LDN193189, activin, and CaCl₂.
[7] A culture method, comprising a step of culturing, in a medium, a cell population containing a parathyroid gland cell in a ratio of 50% or more, and a mesenchymal cell in a ratio of 10% or more.
[8] The culture method according to item 7, wherein the cell population further contains a vascular endothelial cell.
[9] The culture method according to item 7, wherein a matrix composition obtained by embedding the cell population in a matrix is cultured.
[10] The culture method according to item 9, wherein the matrix composition containing the cell population embedded therein is cultured in a state suspended from a breathable membrane.
[11] The culture method according to item 7, wherein the cell population is cultured in a round bottom plate.
[12] The culture method according to item 7, wherein the medium contains two or more components selected from the group consisting of SHH, FGF8, FGF10, Wnt3a, LDN193189, activin, and CaCl₂.
[13] An organoid obtained by the culture method according to any one of items 7 to 12.
[14] A method for producing a three-dimensional organ, comprising a step of performing the culture method according to any one of items 7 to 12.
[15] A three-dimensional organ obtained by the production method according to item 14.
[16] A three-dimensional organ obtained by transplanting, in a non-human animal, the matrix composition according to item 3, or an organoid obtained by the culture method according to any one of items 7 to 12 to be matured.
[17] A method for evaluating a drug or a medicinal agent for treating or preventing a disease related to hyperparathyroidism or hypothyroidism by using the organoid according to item 13, or the three-dimensional organ according to item 15 or 16.
[18] A method for transplanting, in a human or a non-human animal, the organoid according to item 13, or the three-dimensional organ according to item 15 or 16.

### Advantageous Effects of Invention

A cell population of the present invention contains a parathyroid gland cell and a mesenchymal cell respectively in prescribed ratios, and therefore, for example, an organoid excellent in graft survival in transplantation can be produced from such a cell population.

### Brief Description of Drawings

[Figure 1] Figure 1 is a schematic diagram illustrating an example of an embodiment (procedures in an example) of the present invention.
[Figure 2] Figure 2 illustrates an observed image of a parathyroid gland organoid obtained in an example, and a graph of a production amount of parathyroid gland hormone (PTH) obtained by culturing the organoid under environments of different calcium concentrations.
[Figure 3] Figure 3 illustrates immunostained images (with aSMA, PTH, and DAPI) obtained in an example.

### Description of Embodiment

- Definitions -
- Parathyroid Gland Cell

Herein, the term "parathyroid gland cell" refers to a parenchymal cell constituting parathyroid gland. More specifically, the "parathyroid gland cell" is a term encompassing (generically referring to) a main cell and an acidophil. It is noted that a main cell may be changed (transdifferentiated) into an acidophil.

The term "parathyroid gland cell" (each of a parathyroid gland main cell and a parathyroid gland acidophil) used in the present invention encompasses (i) a differentiated mature or terminally differentiated cell having a prescribed function as a parathyroid gland cell (herein, referred to as the "differentiated parathyroid gland cell"), and (ii) a cell having differentiation ability into a parathyroid gland cell, or destined (committed) for differentiation but undifferentiated or in a stage of a stem cell or a parathyroid gland cell and not sufficiently having a prescribed function as a parathyroid gland cell (herein, generically referred to as the "undifferentiated parathyroid gland cell"). The undifferentiated parathyroid gland cell encompasses, for example, a parathyroid gland progenitor cell contained in anterior foregut obtained by differentiation induction from an iPS cell or the like, a foregut endoderm cell, an anterior foregut endoderm cell, and the like.

It can be determined whether or not a cell is a differentiated parathyroid gland cell by determining whether or not expression of one or more mature parathyroid gland markers, such as parathyroid gland hormone (PTH) produced by a mature parathyroid gland main cell, and a calcium ion receptor expressed on a cell surface, are positive (determined as a differentiated parathyroid gland cell when positive). On the other hand, it can be determined whether or not a cell is an undifferentiated parathyroid gland cell by determining whether expression of one or more cell markers, such as CXCR4, EYA1, Six1, and Pax1, are positive or negative.

### - Mesenchymal Cell

The term "mesenchymal cell" (stromal cell) used herein refers to a connective tissue cell present in a connective tissue mainly derived from a mesoderm, and forming a support structure for a cell functioning in the tissue. The term "mesenchymal cell" used herein encompasses both a differentiated cell (differentiated mesenchymal cell) and a cell destined to differentiate into a mesenchymal cell but not yet differentiated into a mesenchymal cell (undifferentiated mesenchymal cell), namely, what is called a mesenchymal stem cell. It is noted that a "vascular endothelial cell" is one of cells differentiated from an undifferentiated mesenchymal cell, but is herein excluded from the definition of the "mesenchymal cell". The undifferentiated mesenchymal cell (stromal cell) also encompasses, for example, a cell present around anterior foregut obtained by differentiation induction from an iPS cell or the like.

It can be determined whether a cell is an undifferentiated mesenchymal cell or a differentiated mesenchymal cell depending on, for example, whether one or more undifferentiated mesenchymal cell markers, such as Stro-1, CD29, CD44, CD73, CD90, CD105, CD133, CD271, and Nestin, are positive or negative (determined as an undifferentiated mesenchymal cell when positive, and determined as a differentiated mesenchymal cell when negative). Besides, it can be determined whether or not a cell is a differentiated mesenchymal (stromal) cell depending on whether or not α-smooth muscle actin (αSMA) is positive (determined as a differentiated mesenchymal (stromal) cell when positive).

### - Vascular Endothelial Cell

The term "vascular endothelial cell" used herein is a term encompassing concepts of both a hemogenic endothelial cell (HEC) and a non-hemogenic endothelial cell (non-HEC). HEC is a vascular endothelial cell capable of producing a hematopoietic stem cell (having hematopoietic ability), and is also designated as a blood cell-producing vascular endothelial cell. On the other hand, non-HEC is a vascular endothelial cell that does not have such hematopoietic ability.

It can be determined whether or not a cell is a vascular endothelial cell by determining whether or not one or more vascular endothelial cell markers, such as TIE2, VEGFR-1, VEGFR-2, VEGFR-3, and CD41, are positive (to be determined as a vascular endothelial cell when positive). Besides, it can be determined whether or not a cell is a differentiated vascular endothelial cell by determining whether or not one or more markers, such as CD31 and CD144, are further positive (to be determined as a differentiated vascular endothelial cell when positive).

It is noted that the term "vascular endothelial cell" used herein can be replaced with a "vascular cell" encompassing a vascular endothelial cell, a vascular smooth muscle cell, and a pericyte.

### - Hemogenic Endothelial Cell (HEC)

The term "hemogenic endothelial cell (HEC)" used herein means a vascular endothelial cell having hematopoietic ability that is CD34-positive and CD73-negative for a cell marker of HEC. The HEC used in the present invention may include a precursor cell thereof. Representative examples of such a precursor cell include cells present in the differentiation process from a precursor cell of a vascular endothelial cell positive for a cell marker Flk-1 (CD309, KDR) (for example, a lateral plate mesoderm cell) to an HEC (see Cell Reports 2, 553-567, 2012). It is noted that a precursor cell obtained at an early stage of differentiation such as one positive for Flk-1 (CD309, KDR) is a precursor cell common between HEC and non-HEC, and hence, the term "HEC precursor cell" encompasses a precursor cell common between HEC and non-HEC unless otherwise stated.

### - Non-hemogenic Endothelial Cell (Non-HEC)

The term "non-hemogenic endothelial cell (non-HEC)" used herein means a vascular endothelial cell that has no hematopoietic ability and is CD31-, CD73- and CD144-positive for cell markers of non-HEC. The non-HEC used in the present invention may include a precursor cell thereof. Representative examples of such a precursor cell include cells present in the differentiation process from a precursor cell of a vascular endothelial cell positive for a cell marker Flk-1 (CD309, KDR) (for example, a lateral plate mesoderm cell) to a non-HEC (see Cell Reports 2, 553-567, 2012). It is noted that a precursor cell obtained at an early stage of differentiation such as one positive for Flk-1 (CD309, KDR) is a precursor cell common between HEC and non-HEC, and hence, the term "non-HEC precursor cell" embraces a precursor cell common between HEC and non-HEC unless otherwise stated.

### - Cell Marker

The term "cell marker" used herein is a (positive marker) gene specifically expressing in a prescribed cell type or a (negative marker) gene not expressing, and specifically refers to a substance generated (positive marker) or not generated (negative marker) as an mRNA through transcription of the gene included in a genome, or as a protein resulting from translation of the mRNA. The cell marker can be preferably labelled (stained) with a fluorescent substance, and is a protein expressed on a cell surface (cell surface marker) easily usable for detection, concentration, isolation and the like of a cell expressing the cell marker.

That a marker gene is "positive" means that expression level of an mRNA or a protein of the gene can be detected by a method usual for or known to those skilled in the art, or is higher than a prescribed threshold value (such as a background level). That a marker gene is "negative" means that the expression level of an mRNA or a protein of the gene cannot be detected by a method usual for or known to those skilled in the art, or is lower than a prescribed threshold value (such as a background level).

It can be determined whether a cell marker is positive or negative by a method usual for or known to those skilled in the art, or based on a qualitative or quantitative result. A cell marker in the form of a protein can be detected or measured for the expression level by utilizing an immunoassay using an antibody specific to the protein, such as ELISA, immunostaining, or flowcytometry. A cell marker in the form of an mRNA can be detected or measured for the expression level by utilizing an assay using a nucleic acid specific to the mRNA, such as RT-PCR (including quantitative PCR), a microarray, or a biochip.

### - Cell Composition (Cell Mixture)

The term "cell composition" used herein refers to a composition that is prepared to be embedded in a matrix (before forming a cell aggregate), and has prescribed cell components (types and ratios) according to the present invention.

### - Cell Aggregate (Spheroid)

The term "cell aggregate" used herein refers to a structure at a previous stage that is prepared from the "cell composition" before being embedded in a matrix, or obtained after embedding the "cell composition" in a matrix and culturing the resultant for a while (before forming an organoid), and does not have a structure or a characteristic as that of an organoid.

### - Organoid

The term "organoid" refers to an artificially created structure (three-dimensional structure) similar to an organ or a tissue. The "organoid" encompasses not only those at a comparatively matured stage as an organoid having functions or structures similar to various organs or tissues, but also those designated as "organ buds" or "anlages" that are at an early-stage of complication. As organoids, not only a "parathyroid gland organoid" corresponding to a target of the present invention (hereinafter sometimes simply referred to as the "organoid") but also various types of organoids of, for example, liver, pancreas, kidney, heart, lung, spleen, esophagus, stomach, thyroid gland, thymus, reproductive gland, brain, and spinal cord are known (for example, see https://www.nejm.org/doi/pdf/10.1056/NEJMra1806175, https://www.nature.com/articles/s41568-018-0007-6, and http://www.amsbio.com/brochures/organoid-culture-handbook.pdf).

The three-dimensional structure of an organoid can be confirmed by visual or microscopic observation. In addition to the confirmation of the three-dimensional structure, the three-dimensional structure can be discriminated by determining whether or not a marker for a cell contained in the organ, particularly a parenchymal cell marker of the organ, is positive, or preferably whether or not a protein of such a marker has been secreted into a culture supernatant. As for the organoid of "parathyroid gland" corresponding to the target of the present invention, for example, parathyroid gland hormone (PTH) can be selected as the cell marker or secretory protein described above.

### - Three-dimensional Organ

The term "three-dimensional organ" refers to a structure that can be designated also as a mature organoid, and contains a cell population or structure more mature than an organoid.

It can be determined whether or not a three-dimensional organ has been obtained from an organoid by making determination from one or more viewpoints such as a density of cells in the structure (whether or not the density is over a prescribed level), a three-dimensional shape of the structure (whether or not the shape is more three-dimensional beyond a prescribed level), function or trait (whether or not a prescribed function or trait, such as metabolic function, has been acquired), and a cell marker (whether or not expression of a gene or protein of a cell marker is positive, whether or not a density of positive cells is over a prescribed level, or whether or not a secretion amount of a marker protein in a culture supernatant is over a prescribed level). The density of the cells, the three-dimensional shape, the function or trait, the cell marker and the like described above can be appropriately set in accordance with the organoid and the three-dimensional organ, and for example, whether or not a level equivalent to or similar to that of an organ in a living body has been achieved can be employed as a criterion for the determination. The three-dimensional organ of "parathyroid gland" corresponding to the target of the present invention can be discriminated, for example, depending on whether or not the structure has a function to adjust a PTH production amount in accordance with a calcium concentration in a culture environment.

The respective cells contained in the cell composition, the cell aggregate and the organoid of the present invention may be derived from a human, or may be derived from an animal except for a human, for example, a mammal such as a mouse, a rat, a dog, a pig, or a monkey. When the organoid is applied to, for example, transplantation in a human, or development of human medicaments (for example, detection of a drug causing drug addiction, which is difficult to find in conventional animal experiments or human cell tests), these cells are preferably derived from a human.

### - Cell Population -

A cell population of the present invention contains a parathyroid gland cell and a mesenchymal cell respectively in prescribed ratios, and may further contain a vascular endothelial cell in an optional ratio if necessary. The term "cell population" used herein encompasses both the "cell composition" and the "cell aggregate" described above. In other words, the "cell population" may refer to the "cell composition" or the "cell aggregate" before being embedded in a matrix in some cases, or may refer to the "cell composition" immediately after being embedded in a matrix, or the "cell composition" obtained by culturing the resultant for a while in other cases. Besides, the term "cell population" used herein may refer to, in a broad sense, a cell population contained in (constituting) the "organoid" and the "three-dimensional organ" described above.

The ratio of the parathyroid gland cell contained in the cell population of the present invention is 50% or more, and preferably 70% or more.

The ratio of the mesenchymal cell contained in the cell population of the present invention is 5% or more, and preferably 10% or more.

When the vascular endothelial cell is used, the ratio of the mesenchymal cell contained in the cell population of the present invention is, for example, 1% or more, and preferably 2% or more.

### - Parathyroid Gland Cell

The "parathyroid gland cell" can encompass a main cell, an acidophil, or both of these, or can encompass a differentiated parathyroid gland cell, an undifferentiated parathyroid gland cell, or both of these. The ratios of the respective cells included in the parathyroid gland cells are arbitrary, and can be appropriately adjusted in accordance with an embodiment.

The parathyroid gland cell may be collected from a living body, or may be produced, by a known method or a method of the present invention, from a pluripotent stem cell such as an ES cell or an iPS cell, or another cell having the ability to differentiate into a parathyroid gland cell.

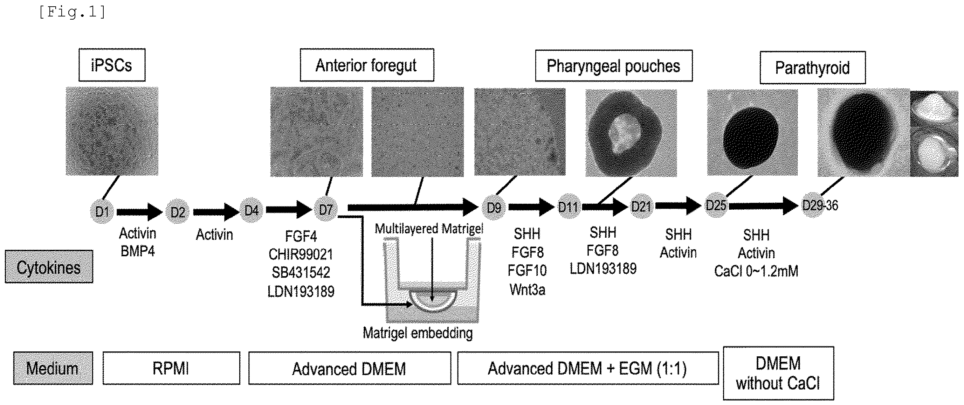

In one preferable embodiment of the present invention, the parathyroid gland cell is contained in a cell aggregate (anterior foregut of Figure 1) obtained by differentiation induction from an iPS cell (or another cell having a prescribed differentiation ability). The parathyroid gland cells in such an embodiment mainly include undifferentiated parathyroid gland cells (parathyroid gland progenitor cells).

### - Mesenchymal Cell

The "mesenchymal cells" can include a differentiated mesenchymal cell, an undifferentiated mesenchymal cell, or both of these. The ratios of the cells included in the mesenchymal cells are arbitrary, and can be appropriately adjusted in accordance with an embodiment.

The mesenchymal cell may be collected from a living body, or may be produced, by a known method or a method of the present invention, from a pluripotent stem cell such as an ES cell or an iPS cell, or another cell having the ability to differentiate into a mesenchymal cell.

In one preferable embodiment of the present invention, the mesenchymal cell is present around a cell aggregate (anterior foregut of Figure 1) containing a parathyroid gland cell obtained by differentiation induction from an iPS cell (or another cell having prescribed differentiation ability). The mesenchymal cells (stromal cells) in such an embodiment mainly include undifferentiated mesenchymal cells.

The cell population of the present invention can be prepared by preparing a parathyroid gland cell and a mesenchymal cell (and a vascular endothelial cell if necessary), and mixing these cells in the prescribed ratios. In one preferable embodiment of the present invention, the cell population is prepared by dissociating once a parathyroid gland cell contained in a cell aggregate obtained from an iPS cell as described above and a mesenchymal cell present therearound by a pipetting operation or the like, and mixing these cells so that the numbers of the cells meet the above-described prescribed ratios.

### - Vascular Endothelial Cell

The "vascular endothelial cell" can encompass a hemogenic endothelial cell (HEC), a non-hemogenic endothelial cell (non-HEC), or both of these. The ratios of the respective cells included in the vascular endothelial cells are arbitrary, and can be appropriately adjusted in accordance with an embodiment.

The vascular endothelial cell may be a cell collected from a living body (such as a microvessel endothelial cell (MVEC), or an umbilical-vein endothelial cell (UVEC)), or may be a cell obtained by differentiating a pluripotent stem cell such as an ES cell or an iPS cell, or another cell having the ability to differentiate into a vascular endothelial cell.

The hemogenic endothelial cell (HEC) may be a cell collected from a living body, or may be a cell obtained by differentiating, by a known method, a pluripotent stem cell such as an ES cell or an iPS cell, or another cell having the ability to differentiate into a vascular endothelial cell (such as a lateral mesodermal cell). For example, a method for producing an HEC from an iPS cell can be performed by referring to PLoS One, 2013; 8(4): e59243, Nat Biotechnol. 2014; 32(6): 554-61, Sci Rep. 2016; 6: 35680, or the like.

The non-hemogenic endothelial cell (non-HEC) may be a cell collected from a living body, or may be a cell obtained by differentiating, by a known method, a pluripotent stem cell such as an ES cell or an iPS cell, or another cell having the ability to differentiate into a vascular endothelial cell (such as a lateral mesodermal cell). For example, a method for producing a non-HEC from an iPS cell can be performed by referring to Nat Cell Biol. 2015; 17(8): 994-1003, Cell Rep. 2017; 21(10): 2661-2670, or the like.

In one preferable embodiment of the present invention, the vascular endothelial cell is an HEC or non-HEC obtained by differentiation induction from an iPS cell (or another cell having prescribed differentiation ability).

### - Matrix Composition -

A matrix composition of the present invention contains (1) a matrix, and (2) the cell composition of the present invention described above.

As the "matrix" of the present invention, a general extracellular matrix that is a solid at room temperature or more, and is used in cell culture, particularly in three-dimensional cell culture can be used. An extracellular matrix basically contains fibrous protein and proteoglycan as principal components, and examples of such components include elastin, entactin, osteonectin, collagen (type IV collagen), tenascin, thrombospondin, perlecan, vitronectin, fibrillin, fibronectin, heparin (sulfate), and laminin. For example, a basement membrane matrix known as a trade name "Matrigel" (Corning), containing laminin, collagen (type IV collagen) and entactin, and also containing growth factors such as EGF, IGF-1, PDGF, and TGF-β is one of preferable matrixes used in the present invention. Besides, a self-assembling peptide known as a hydrogel, such as hydrogels containing arginine, glycine and asparagine, can be used as the matrix of the present invention. As the matrix, one of these may be used, or two or more of these may be used in the form of a mixture, or without mixing (so as to form different layers when solidified).

Those skilled in the art can appropriately adjust the composition and concentration (undiluted or diluted) of the matrix so that the resultant matrix having the cell population embedded therein have appropriate hardness. For example, the matrix (for example, Matrigel) can be used in the form of a mixture with a liquid medium for improving handleability, and attaining appropriate hardness when mixed with the cell population and solidified (in a state where the cell population is embedded therein). In such an embodiment, the liquid medium to be mixed with the matrix can be the same as a liquid medium used for culturing the cell population embedded therein by immersing the matrix composition therein in "Culture System" and "Culture Method" of the present invention.

The matrix composition can be prepared generally by adding an appropriate amount of the matrix containing a suitable component to a liquid medium containing the cell population, and solidifying the resultant matrix (with an ionic solution or an ionic molecule for solidifying a hydrogel added if necessary). For example, a matrix that is in a liquid state at 4°C or less (such as Matrigel) and the cell population are mixed by stirring, the resultant is allowed to stand still at 37°C or more to solidify the matrix, and thus, the matrix composition is obtained. When the matrix is added to a comparatively large amount of medium in which the cell population is suspended, a sufficient amount of the matrix is added to attain sufficient hardness of the matrix, and thus, the cell population is properly held within the matrix without submerging onto the bottom. The hardness of the matrix can be adjusted, for example, in a range of 0.05 to 50 kPa.

Application of the matrix composition of the present invention is not especially limited, and the matrix composition can be used, for example, producing a parathyroid gland organoid (and furthermore, a three-dimensional organ of parathyroid gland) by a culture method of the present invention.

Besides, the matrix composition of the present invention can be used for producing a non-human chimeric animal by transplanting the matrix composition in a non-human animal (such as a mouse, a rabbit, a pig, a dog, or a monkey) to be differentiated and matured therein to a parathyroid gland organ (and further a three-dimensional organ of parathyroid gland). In such an embodiment, the matrix composition may be transplanted after being scraped from a breathable membrane on which it has been formed, or may be transplanted in a state where it is formed on a breathable membrane made of a biodegradable material.

In one embodiment of the present invention, the matrix composition may have a multilayer structure. An example of the multilayer structure includes a structure including a first matrix and a second matrix, in which the first matrix wraps around the second matrix, and the first matrix has at least one opening. Compositions of the first matrix and the second matrix may be the same or different. The respective cells contained in the cell population of the present invention may be contained in the same matrix, or may be contained in the different matrixes. For example, when the vascular endothelial cell (vascular cell) is added separately to a matrix different from a matrix containing the other cells, an organoid having blood vessels (vascular channels) having a hierarchical structure can be produced. Either of the first matrix and the second matrix may not contain cells. It is noted that the "opening" of the first matrix refers to a portion where the second matrix is not wrapped. In other words, the second matrix can receive, through the "opening" without via the first matrix, oxygen, nutrients, and other substances necessary for cells contained in the second matrix from the outside. A matrix composition having the above-described multilayer structure can be produced by a known method, and when the matrix composition is formed on, for example, a breathable membrane (described in detail below), a method in which the second matrix is dropped onto the breathable membrane and then solidified, and the first matrix containing a cell if necessary is dropped onto the solidified second matrix and then solidified can be employed. In this production method, a portion where the second matrix and the breathable membrane are in contact with each other is not covered with the first matrix, and corresponds to the "opening".

### - Culture System -

A culture system of the present invention includes the cell population or the matrix composition of the present invention described above, a medium for culturing the cell population, and a culture vessel. It is noted that the system of the present invention is representatively used for performing the culture method of the present invention described below, and the technical items related to the "medium" and the "culture vessel" defined in the system of the present invention can be applied as technical items related to a "medium" and a "culture vessel" defined (used) in the culture method of the present invention. On the contrary, the "matrix composition" of the culture system of the present invention can be a composition in a "state suspended from a breathable membrane" described below regarding the culture method of the present invention.

### - Medium

The "medium" is usually a liquid medium, and a suitable medium in accordance with the cell population or the matrix composition (the cell population embedded therein) may be used. In general, a mixture of media suitable for culturing the respective cells contained in the cell population can be used as the medium of the culture system of the present invention. The media suitable for culturing the parathyroid gland cell and the mesenchymal cell contained in the cell population of the present invention, and the vascular endothelial cell contained if necessary, are known, and suitable media are selected from the known media to be mixed in appropriate ratios, and thus, the medium of the culture system of the present invention can be prepared.

Examples of a basal medium for the parathyroid gland cell include RPMI (Fujifilm Corporation), DMEM (Gibco), and EGM (Lonza). Examples of an additive for the parathyroid gland cell include one or more selected from the group consisting of activin A, BMP4, FGF4, a GSK3 inhibitor (CHIR99021), a TGFb/Smad signaling pathway inhibitor (SB431542), a BMP signaling pathway inhibitor (LDN193189), SHH, FGF8, FGF10, Wnt3, and CaCl₂. As the medium for the parathyroid gland cell, a medium obtained by precedently adding a prescribed component to the basal medium, such as Advanced DMEM (Gibco), may be also used.

Examples of a basal medium for a vascular endothelial cell include DMEM/F-12(Gibco), Stempro-34 SFM (Gibco), Essential 6 medium (Gibco), Essential 8 medium (Gibco), EGM (Lonza), BulletKit (Lonza), EGM-2 (Lonza), BulletKit (Lonza), EGM-2 MV (Lonza), VascuLife EnGS Comp Kit (LCT), Human Endothelial-SFM Basal Growth Medium (Invitrogen), and Human Microvascular Endothelial Cell Growth Medium (TOYOBO). Examples of an additive for a vascular endothelial cell include one or more selected from the group consisting of B27 Supplements (Gibco), BMP4 (bone morphogenetic protein 4), GSK β inhibitor (such as CHIR99021), VEGF (vascular endothelial cell growth factor), FGF2 (fibroblast growth factor (also designated as bFGF (basic fibroblast growth factor))), Folskolin, SCF (stem cell factor), TGF β receptor inhibitor (such as SB431542), Flt-3L (Fms-related tyrosine kinase 3 ligand), IL-3 (interleukin 3), IL-6 (interleukin 6), TPO (thrombopoietin), hEGF (recombinant human epithelial cell growth factor), hydrocortisone, ascorbic acid, IGF1, FBS (fetal bovine serum), an antibiotic (such as gentamicin or amphotericin B), heparin, L-glutamine, phenol red and BBE.

In one embodiment of the present invention, the medium contains two or more components selected from the group consisting of SHH, FGF8, FGF10, Wnt3a, BMPi, activin, and CaCl₂. A combination of these components can be appropriately adjusted in accordance with a culture stage of the cell population of the present invention (such as the number of days from start of the culture, and properties of the cell population). For example, the above-described components can be added to the medium in any of the following combinations:
[i] SHH, FGF8, FGF10, and Wnt3a;
[ii] SHH, FGF8, and BMPi;
[iii] SHH and activin; and
[iv] SHH, activin and CaCl₂.

### - Culture Vessel

The "culture vessel" (cell culture device) is not especially limited as long as the cell population or the matrix composition (cell population embedded therein) can be cultured therein. Various culture vessels (or culture systems) for producing an organoid, an artificial organ or the like from various cell populations are known, and these can be used, or a similar vessel suitably matched to the present invention can be used also in the present invention. For example, the culture vessel may be made of a material to which the cell population or the matrix may not attach, or may be surface treated. When the cell population in a state embedded in the matrix composition is to be cultured, a plate including one or more wells according to the shape or size of the matrix composition (which may be in a state suspended from a breathable membrane as described below) can be used. When the cell population not in a state embedded in the matrix composition is to be cultured, for example, a round bottom plate (Corning Elplasia or the like) can be used to form a cell aggregate. Alternatively, various culture vessels for suspension culture, stirred-suspension culture (such as a spinner flask), channel culture (such as organ-on-chip) can be used.

The system of the present invention can further include, in addition to the above-described constituting elements, desired constituting elements in accordance with an embodiment, such as a tool for holding a breathable membrane (described in detail below) in a state where the matrix composition is suspended therefrom, a culture device for performing suspension culture, stirred-suspension culture or the like, and a culture device for performing culture in appropriate atmosphere and temperature.

### - Culture Method -

The culture method of the present invention includes a step of culturing, in a medium, the cell population of the present invention described above, or the matrix composition of the present invention obtained by embedding the cell population in a matrix.

### - Breathable Membrane

In one preferable embodiment of the present invention, the matrix composition is cultured in a state suspended from a breathable membrane.

The term "breathable membrane" refers to a membrane having at least oxygen permeability, and further having, if necessary, permeability of carbon dioxide or another desired gas. Various breathable membranes are known, and examples include membranes made of fibers of polyethylene terephthalate (PET), polydimethylsiloxane (PDMS), fluorocarbon, polytetrafluoroethylene (PTFE), polyurethane and the like, or a biodegradable or bioabsorbable material such as collagen. The breathable membrane may be, if necessary, subjected to a surface treatment for increasing or reducing cell attachment, such as a coating treatment with an ECM of collagen or the like. Alternatively, the breathable membrane may be a membrane obtained by laminating a breathable membrane on a porous membrane (mesh) made of a fiber different from the breathable membrane (hybrid membrane) if necessary.

The matrix composition "in a state suspended from a breathable membrane" can be produced by dropping the matrix composition before solidification onto the breathable membrane, solidifying the resultant, and inverting the resultant in such a manner as to cause the solidified matrix composition attached to the breathable membrane to face downward (project downward). When the breathable membrane having the matrix composition suspended therefrom is immobilized in an upper portion of the culture vessel with an appropriate member such as a holder, although the matrix composition is immersed in the medium, the breathable membrane is not immersed but placed in the air (or in a desired culture atmosphere), and thus, the cell population (the cell composition, the cell aggregate, the organoid, or the like) embedded in the matrix can be cultured with gas exchange through the breathable membrane ensured.

The composition of the medium and various culture conditions (such as an atmosphere, a temperature, and a period) employed in the culture method of the present invention can be appropriately adjusted in accordance with the purposes. In a representative embodiment, the culture method of the present invention can be performed, until an organoid is formed from the cell population (cell composition or cell aggregate), in a medium containing suitable components (with the composition of the medium changed if necessary) for a sufficient time period (of, for example, 1 to 10 days) at an appropriate temperature (of, for example, 30 to 40°C, and preferably about 37°) in an appropriate CO₂ concentration (of, for example, 5%).

The culture method of the present invention is representatively a method for producing an organoid of parathyroid gland. An organoid of the present invention is one obtained by the culture method of the present invention.

The culture method of the present invention can be alternatively performed for producing a three-dimensional organ of parathyroid gland (as a part of the production method). The organoid of the present invention is one obtained by a production method including a step of performing the culture method of the present invention.

The composition of the medium and various culture conditions (such as an atmosphere, a temperature, and a period) to be employed in the method for producing a three-dimensional organ of the present invention can be basically the same as those employed in the culture method of the present invention, and can be appropriately adjusted if necessary, for example, a culture period sufficient for forming a three-dimensional organ can be employed. For example, when a three-dimensional organ of parathyroid gland is to be produced, culture is performed for 20 to 40 days from a day when culture of the cell population is started, or culture is performed for another 10 to 20 days for mature from a day when formation of a three-dimensional structure as a parathyroid gland organoid can be confirmed.

When the organoid of parathyroid gland obtained from the cell population or the matrix composition of the present invention, or obtained by the culture method of the present invention is transplanted in a non-human animal for mature, a three-dimensional organ can be obtained. The organoid and the three-dimensional organ of parathyroid gland of the present invention can be transplanted in a human or a non-human animal.

An artificial organ can be produced from a three-dimensional organ by linkage to an extracorporeal circulation. Such an artificial organ can be used as an organ failure model, or can be used for evaluating the function of an organ. As for such applications, for example, WO2013/047720 and the like can be referred to.

The organoid and the three-dimensional organ of the present invention obtained as described above can be used for transplantation in a human or a non-human animal, for example, for treating or preventing a disease related to hyperparathyroidism or hypothyroidism. Examples of the "disease related to hyperparathyroidism or hypothyroidism" include primary hyperparathyroidism, secondary hyperparathyroidism, and hypoparathyroidism.

The organoid, the three-dimensional organ, and the non-human chimeric animal of the present invention obtained as described above can be used for performing various evaluation methods of, for example, prediction of a drug metabolism profile in a human, evaluation of a medicinal effect, evaluation of toxicity, and evaluation of drug interaction of a medicinal agent (in a formulated form) or a drug (in a form before formulation, active ingredient) for treating or preventing the disease related to hyperparathyroidism or hypothyroidism. The "drug" is not especially limited as long as it acts on the organoid, the three-dimensional organ, or the non-human chimeric animal, or it is used for analyzing whether or not it acts, and can be selected in accordance with the purpose. The "drug" is not especially limited as long as it can be used as an active ingredient of a medicament, such as a low molecular weight medicament, an antibody medicament, a peptide medicament, or a nucleic acid medicament. Besides, the "medicinal agent" is obtained by formulation by a general method with the "drug" used as an active ingredient in combination with an additive or the like used in accordance with a desired dosage form.

More specifically, a drug evaluation method using the organoid or three-dimensional organ of the present invention *in vitro* includes, for example, a step of culturing the organoid or three-dimensional organ in a culture fluid supplemented with a drug to be evaluated, and a step of evaluating a desired item of the drug, such as efficacy or safety, against the organoid or three-dimensional organ. Alternatively, a drug evaluation method using a non-human chimeric animal provided with the three-dimensional organ of the present invention *in vivo* includes, for example, a step of administering a drug to be evaluated to the non-human chimeric animal, and a step of evaluating a desired item of the drug, such as efficacy or safety, against (the three-dimensional organ provided in) the non-human chimeric animal.

### Examples

### [1] Process for Producing Anterior Foregut from Human iPS Cell

Human iPS cells (1383D2 or 511-3E; Center for iPS Cell Research and Application, Kyoto University) were seeded in a 24-well plate coated with 0.5 µg/mL iMatrix511 at 1.5 to 1.8 x 10⁵ cells/well, cultured in AK02N (Ajinomoto Co., Ltd.) (8 ml) supplemented with a ROCK inhibitor (Y-27632, Wako) (10 µM) in 5% CO₂ at 37°C for 24 hours, and then further cultured for 1 day with the medium replaced with AK02N not supplemented with the ROCK inhibitor (D0 to D1). The medium was replaced with one obtained by adding activin A (100 ng/mL) and BMP4 (50 ng/mL) to a basal medium obtained by adding, to RPMI (Fujifilm Corporation) (2 ml), 1% Penicillin-Streptomycin and HEPES (10 mM) (both Gibco) (hereinafter referred to as the basal medium A), and the resultant was cultured in 5% CO₂ at 37°C for 1 day (D1 to D2). The medium was replaced with one obtained by adding, to the basal medium A (2 ml), activin A (100 ng/mL) and 0.2% FBS gold (MP Biomedicals), the resultant was cultured in 5% CO₂ at 37°C for 1 day, and the medium was further replaced with a similar medium obtained with the amount of FBS gold to be added changed to 2%, followed by culturing for another 1 day (D2 to D4). The medium was replaced with a medium obtained by adding, to Advanced DMEM (Gibco) (2 ml), 1% Penicillin-Streptomycin, HEPES (10 mM), 2% B27, 1% N2, and 1% GlutaMAX (all Gibco) (hereinafter referred to as the basal medium B) supplemented with FGF4 (100 ng/mL), a GSK3 inhibitor (CHIR99021) (1 µM), a TGFb/Smad signaling pathway inhibitor (SB431542) (5 µM) and a BMP signaling pathway inhibitor (LDN193189) (5 µM) (hereinafter referred to as the "anterior foregut medium"), and the resultant was cultured in 5% CO₂ at 37°C for 3 days (D4 to D7) to obtain an anterior foregut cell population containing a mesenchymal cell.

### [2] Process for Preparing Cell Population and Process for Producing Matrix Composition

The anterior foregut cell population containing a mesenchymal cell obtained as described in [1] above was dissociated by a pipetting operation, and the resultant was recovered into a 1.5 mL tube, and such a pipetting operation was repeated about 10 to 20 times for separation into a cramp or spheroid of cells of 20 to 100 µm, and thus, an anterior foregut cell and a mesenchymal cell were isolated.

A matrix was prepared by mixing the anterior foregut medium and Matrigel (BD Pharmingen) in a volume ratio of 1:1 (hereinafter referred to as the "matrix preparation").

The matrix preparation was dropped in an amount of 15 µL onto an inverted Hanging Drop Insert (Millipore), and the resultant was solidified by increasing the temperature to 37°C to form a "second matrix layer".

The isolated parathyroid gland cell and mesenchymal cell were mixed in the matrix preparation at 4°C. The thus obtained mixture was dropped in an amount of 9 µL onto the second matrix layer formed as described above, and the resultant was solidified by increasing the temperature to 37°C to form a "first matrix layer".

### [3] Process for Producing Organoid

To a low-attachment 24-well plate, 600 µL of the anterior foregut medium was added. A matrix composition including the first and second matrix layers formed on the Hanging Drop Insert as described above was inserted toward the medium in the well, and the resultant was cultured in 5% CO₂ at 37°C for 2 days (D7 to D9).

The medium was replaced with a medium obtained by mixing the basal medium B and EGM (Lonza) in a volume ratio of 1:1 (hereinafter referred to as the "parathyroid gland organoid basal medium") supplemented with SHH (50 ng/mL), FGF8 (50 ng/mL), FGF10 (50 ng/mL) and Wnt3a (50 ng/mL), and the resultant was cultured in 5% CO₂ at 37°C for 2 days (D9 to D11) to obtain a pharyngeal pouch.

The medium was replaced with a medium obtained by supplementing the organoid basal medium with SHH (100 ng/mL), FGF 8 (50 ng/mL) and LDN193189 (5 µM), and the resultant was cultured in 5% CO₂ at 37°C for 10 days (D11 to D21).

The medium was replaced with a medium obtained by supplementing the organoid basal medium with SHH (100 ng/mL) and activin (100 ng/mL) (hereinafter referred to as the "parathyroid gland organoid maturing medium"), and the resultant was cultured in 5% CO₂ at 37°C for 4 days (D21 to D25) for maturation to a parathyroid gland organoid.

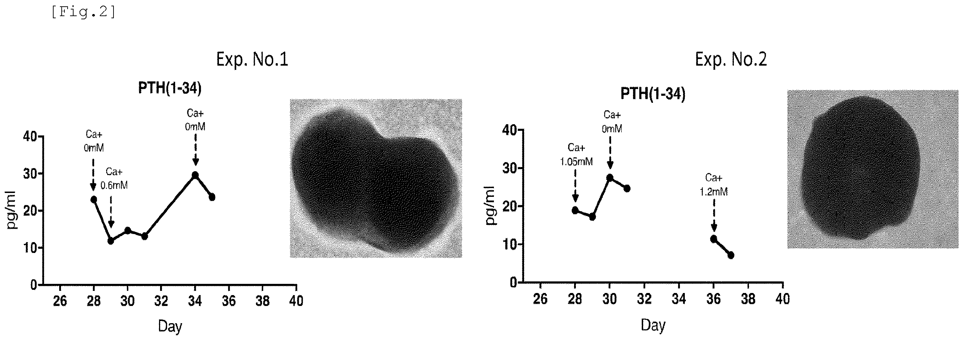

The medium was replaced with a medium obtained by supplementing, with SHH (100 ng/mL) and activin (100 ng/mL), a basal medium obtained by adding, to a calcium-free DMEM (Gibco), 1% Non-Essential Amino Acid solution, Sodium Pyruvate (1 mM) (both Gibco), 1% Penicillin-Streptomycin, HEPES (10 mM), 2% B27, 1% N2, and 1% GlutaMAX (hereinafter referred to as the "parathyroid gland organoid calcium-free medium"), and the resultant was cultured in 5% CO₂ at 37°C for 2 days (D25 to D27) to urge production of parathyroid gland hormone (PTH) in the parathyroid organoid. Besides, the medium was replaced with the parathyroid gland organoid calcium-free medium, or a medium obtained by adding CaCl₂ in a concentration of 0.6 to 1.2 mM to this medium, the resultant was cultured under the same conditions for 1 day, and then, 1 mL of a culture supernatant was recovered for collecting a culture supernatant for PTH measurement. This operation was successively performed from D28 to D37 using media having different calcium concentrations, and thus, culture supernatants derived from the same organoid resulting from culture under environments of different calcium concentrations were collected. The culture supernatants obtained from D28 to D37 were used to measure the production amount of parathyroid gland hormone on the N-terminal side (1 to 34) by competitive EIA. Results are illustrated in Figure 2.

After the medium was replaced with the parathyroid gland organoid calcium-free medium, the Hanging Drop Insert to which the matrix composition containing the organoid having been cultured in 5% CO₂ at 37°C until D35 had been attached was taken out, and the organoid was scraped with a scraper. The resultant organoid was put in a 2 mL tube containing a 4% paraformaldehyde/PBS immobilization solution, and immobilized by allowing it stand still at 4°C for 2 hours. After the immobilization, the immobilization solution was removed to be replaced with PBS.

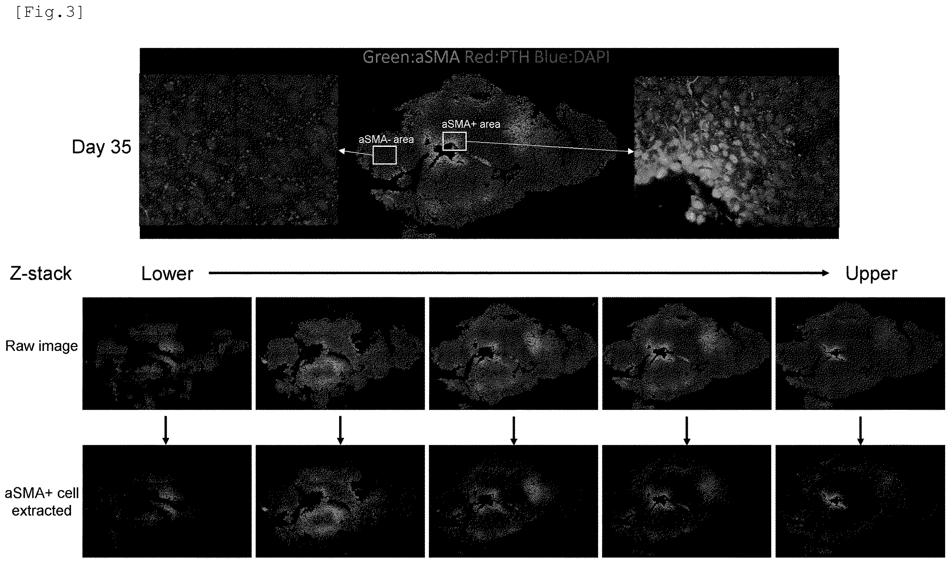

The immobilized organoid was subjected to a transparency treatment and immunofluorescence staining with SCALEVIEW-S (FUJIFILM Wako Pure Chemical Corporation). As the method, AbSca/e method was employed (https://labchem-wako.fujifilm.com/jp/category/00593.html). For the immunofluorescence staining, primary antibodies: an anti-alpha smooth muscle Actin (aSMA) antibody (Abeam, ab7817), an anti-PTH antibody (Abeam, ab166631), and fluorescence labeling secondary antibodies binding to these antibodies (Novex Donkey anti-Mouse IgG (H + L) Secondary Antibody, Novex Donkey anti-Rabbit IgG (H + L) Secondary Antibody (Invitrogen)), and a nuclear staining reagent (DAPI) were used. The thus obtained stained specimen was subjected to fluorescence image observation with LSM 880 Confocal Laser Microscope (Zeiss), and the obtained fluorescence images were analyzed with imaging software Zeiss ZEN (Zeiss). An aSMA positive rate corresponding to an abundance ratio of stromal cells was calculated by extracting, with Colocalization function of Zeiss ZEN, cells having an aSMA fluorescence intensity equal to or higher than a prescribed level from respective DAPI-labeled cells, and quantitatively determining areas of the extracted cells. The immunostained images are illustrated in Figure 3, and results of the quantitative determination is shown in Table 1.

**[Table 1]**

| * Unit = Pixel | | | | | | |
|---|---|---|---|---|---|---|
| Raw image | | | | | | |
| Z-stack No. | 1 | 2 | 3 | 4 | 5 | Total |
| Background Global | 6594770 | 6892833 | 7164873 | 8545915 | 11184365 | 40382756 |
| | 14146560 | 14146560 | 14146560 | 14146560 | 14146560 | 70732800 |
| Cell Area | 7551790 | 7253727 | 6981687 | 5600645 | 2962195 | 30350044 |

| | | | Cell Area = Global - Background | | | |
|---|---|---|---|---|---|---|
| aSMA+ cell Extracted | | | | | | |
| Z-stack No. | 1 | 2 | 3 | 4 | 5 | Total |
| Background | 13276997 | 12661459 | 12051948 | 11797158 | 13011931 | 62799493 |
| Global | 14146560 | 14146560 | 14146560 | 14146560 | 14146560 | 70732800 |
| Cell Area | 869563 | 1485101 | 2094612 | 2349402 | 1134629 | 7933307 |
| | | Cell Area = Global - Background | | | | |
| aSMA+ % 26.14% | | | | | | |
| aSMA+ % = Cell Area(aSMA+ cell extracted) / Cell Area(Raw image) | | | | | | |

| | | | | | | |
|---|---|---|---|---|---|---|
| | | | | | | |

## Claims

1. A cell population, comprising a parathyroid gland cell in a ratio of 50% or more, and a mesenchymal cell in a ratio of 10% or more.

2. The cell population according to claim 1, further comprising a vascular endothelial cell.

3. A matrix composition, comprising a matrix, and the cell population according to claim 1 in a state embedded in the matrix.

4. A culture system, comprising the cell population according to claim 1 or the matrix composition according to claim 3, a medium for culturing the cell population, and a culture vessel.

5. The culture system according to claim 4, wherein the culture vessel is a round bottom plate.

6. The culture system according to claim 4, wherein the medium contains two or more components selected from the group consisting of SHH, FGF8, FGF10, Wnt3a, LDN193189, activin, and CaCl₂.

7. A culture method, comprising a step of culturing, in a medium, a cell population containing a parathyroid gland cell in a ratio of 50% or more, and a mesenchymal cell in a ratio of 10% or more.

8. The culture method according to claim 7, wherein the cell population further contains a vascular endothelial cell.

9. The culture method according to claim 7, wherein a matrix composition obtained by embedding the cell population in a matrix is cultured.

10. The culture method according to claim 9, wherein the matrix composition containing the cell population embedded therein is cultured in a state suspended from a breathable membrane.

11. The culture method according to claim 7, wherein the cell population is cultured in a round bottom plate.

12. The culture method according to claim 7, wherein the medium contains two or more components selected from the group consisting of SHH, FGF8, FGF10, Wnt3a, LDN193189, activin, and CaCl₂.

13. An organoid obtained by the culture method according to any one of claims 7 to 12.

14. A method for producing a three-dimensional organ, comprising a step of performing the culture method according to any one of claims 7 to 12.

15. A three-dimensional organ obtained by the production method according to claim 14.

16. A three-dimensional organ obtained by transplanting, in a non-human animal, the matrix composition according to claim 3, or an organoid obtained by the culture method according to any one of claims 7 to 12 to be matured.

17. A method for evaluating a drug or a medicinal agent for treating or preventing a disease related to hyperparathyroidism or hypothyroidism by using the organoid according to claim 13, or the three-dimensional organ according to claim 15 or 16.

18. A method for transplanting, in a human or a non-human animal, the organoid according to claim 13, or the three-dimensional organ according to claim 15 or 16.
